# EUROPEAN PATENT SPECIFICATION

(11) **EP 2 066 605 B1**
(45) Date of publication and mention of the grant of the patent: **25.12.2013**
(21) Application number: 07837712.4
(22) Date of filing: 05.09.2007
(51) Int. Cl.: C07C 17/354, C07C 21/18, C07C 17/25

(54) **PROCESS TO MANUFACTURE 2,3,3,3-TETRAFLUOROPROPENE**
VERFAHREN ZUR HERSTELLUNG VON 2,3,3,3-TETRAFLUORPROPEN
PROCÉDÉ DE FABRICATION DU 2,3,3,3-TÉTRAFLUOROPROPÈNE

(30) Priority: 05.09.2006 US 842585 P
(43) Date of publication of application: 10.06.2009
(73) Proprietor: E.I. DU PONT DE NEMOURS AND COMPANY, Wilmington, DE 19898 (US)
(72) Inventor: RAO, Velliyur Nott Mallikarjuna, Wilmington, DE 19809 (US); SIEVERT, Allen Capron, Elkton, MD 21921 (US); NAPPA, Mario Joseph, Newark, DE 19711 (US)
(74) Representative: Matthews, Derek Peter
(86) International application number: PCT/US2007/019315
(87) International publication number: WO 2008/030440

(56) References cited:
- EP-A- 0 974 571
- US-A- 5 986 151
- US-A- 6 031 141
- US-B1- 7 026 520
- KNUNYANTS I L ET AL: "REACTIONS OF FLUORO OLEFINS COMMUNICATION 13. CATALYTIC HYDROGENATION OF PERFLUORO OLEFINS" 1960, BULLETIN OF THE ACADEMY OF SCIENCES OF THE USSR, DIVISION OF CHEMICAL SCIENCES, PAGE(S) 1312-1317 , XP000578879 ISSN: 0568-5230 the whole document

## Description

### BACKGROUND INFORMATION

### Field of the Disclosure

This disclosure relates in general to processes for the production of fluorinated olefins.

### Description of the Related Art

The refrigeration industry has been working for the past few decades to find replacement refrigerants for the ozone depleting chlorofluorocarbons (CFCs) and hydrochlorofluorocarbons (HCFCs) being phased out as a result of the Montreal Protocol. The solution for most refrigerant producers has been the commercialization of hydrofluorocarbon (HFC) refrigerants. The new HFC refrigerants, HFC-134a being the most widely used at this time, have zero ozone depletion potential and thus are not affected by the current regulatory phase-out as a result of the Montreal Protocol.

In addition to ozone depleting concerns, global warming is another environmental concern. Thus, there is a need for heat transfer compositions that have not only low ozone depletion potentials, but also low global warming potentials. Certain hydrofluoroolefins meet both goals. Thus there is a need for manufacturing processes that provide halogenated hydrocarbons and fluoroolefins that contain no chlorine and also have lower global warming potential than current commercial refrigeration products. HFC-1234yf is one such hydrofluoroolefin.

### SUMMARY

Disclosed is a process for producing CF₃CF=CH₂ comprising the steps of (a) adding hydrogen and CF₃CF=CHF to a reaction vessel containing a hydrogenation catalyst; (b) reacting said CF₃CF=CHF with hydrogen over said hydrogenation catalyst to produce CF₃CHFCH₂F; and (c) dehydrofluorinating CF₃CHFCH₂F in the vapor phase over a catalyst selected from the group consisting of aluminum fluoride; gamma alumina, fluorided alumina; metals on aluminum fluoride; metals on fluorided alumina; oxides, fluorides, and oxyfluorides of magnesium, zinc and mixtures of magnesium and zinc and/or aluminum; lanthanum oxide and fluorided lanthanum oxide; chromium oxides, fluorided chromium oxides, and cubic chromium trifluoride; carbon, acid-washed carbon, activated carbon, three dimensional matrix carbonaceous materials; and metal compounds supported on carbon, to produce CF₃CF=CH₂. The metal compounds are oxides, fluorides, and oxyfluorides of at least one metal selected from the group consisting of sodium, potassium, rubidium, cesium, yttrium, lanthanum, cerium, praseodymium, neodymium, samarium, chromium, iron, cobalt, rhodium, nickel, copper, zinc, and mixtures thereof.

Also disclosed is a process for producing CF₃CF=CH₂ comprising the steps of (a) adding hydrogen and CF₃CF=CHF to a reaction vessel containing a hydrogenation catalyst; (b) reacting said CF₃CF=CHF with hydrogen over said hydrogenation catalyst to produce CF₃CHFCH₂F; and and (c) dehydrofluorinating CF₃CHFCH₂F in the vapor phase over a catalyst selected from the group consisting of aluminum fluoride; gamma alumina, fluorided alumina; oxides, fluorides, and oxyfluorides of magnesium, zinc and mixtures of magnesium and zinc and/or aluminum; lanthanum oxide and fluorided lanthanum oxide; chromium oxides, fluorided chromium oxides, and cubic chromium trifluoride; carbon, acid-washed carbon, activated carbon, three dimensional matrix carbonaceous materials; to produce CF₃CF=CH₂.

Also disclosed is a process for producing CF₃CF=CH₂ comprising the steps of (a) adding hydrogen and CF₃CF=CHF to a reaction vessel containing a hydrogenation catalyst; (b) reacting said CF₃CF=CHF with hydrogen over said hydrogenation catalyst to produce CF₃CHFCH₂F; and (c) dehydrofluorinating CF₃CHFCH₂F by reaction with a basic aqueous solution in the presence of a non-aqueous, non-alcoholic solvent, and in the presence of a phase transfer catalyst.

The foregoing general description and the following detailed description are exemplary and explanatory only and are not restrictive of the invention, as defined in the appended claims.

### DETAILED DESCRIPTION

Many aspects and embodiments have been described above and are merely exemplary and not limiting.

Other features and benefits of any one or more of the embodiments will be apparent from the following detailed description, and from the claims

Before addressing details of embodiments described below, some terms are defined or clarified.

As used herein, the basic aqueous solution is a liquid (whether a solution, dispersion, emulsion, or suspension and the like) that is primarily an aqueous liquid having a pH of over 7. In some embodiments the basic aqueous solution has a pH of over 8. In some embodiments, the basic aqueous solution has a pH of over 10. In some embodiments, the basic aqueous solution has a pH of 10-13. In some embodiments, the basic aqueous solution contains small amounts of organic liquids which may be miscible or immiscible with water. In some embodiments, the liquid medium in the basic aqueous solution is at least 90% water. In one embodiment the water is tap water; in other embodiments the water is deionized or distilled.

The base in the aqueous basic solution is selected from the group consisting of hydroxide, oxide, carbonate, or phosphate salts of alkali metals, alkaline earth metals, and mixtures thereof. In one embodiment, bases which may be used include lithium hydroxide, sodium hydroxide, potassium hydroxide, calcium hydroxide, magnesium oxide, calcium oxide, sodium carbonate, potassium carbonate, sodium phosphate, potassium phosphate, or mixtures thereof.

In certain embodiments, the non-aqueous non-alcoholic solvent is selected from the group consisting of alkyl and aryl nitriles, alkyl and aryl ethers, amides, ketones, sulfoxides, phosphate esters and mixtures thereof.

As used herein, phase transfer catalyst is intended to mean a substance that facilitates the transfer of ionic compounds into an organic phase from an aqueous phase or from a solid phase. The phase transfer catalyst facilitates the reaction of these dissimilar and incompatible components. While various phase transfer catalysts may function in different ways, their mechanism of action is not determinative of their utility in the present invention provided that the phase transfer catalyst facilitates the dehydrofluorination reaction.

The phase transfer catalyst is selected from the group consisting of crown ethers, onium salts, cryptands, polyalkylene glycols, and mixture thereof.

As used herein, cryptands are any of a family of bi- and polycyclic multidentate ligands for a variety of cations.

As used herein, the terms "comprises," "comprising," "includes," "including," "has," "having" or any other variation thereof, are intended to cover a non-exclusive inclusion. For example, a process, method, article, or apparatus that comprises a list of elements is not necessarily limited to only those elements but may include other elements not expressly listed or inherent to such process, method, article, or apparatus. Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

Also, use of "a" or "an" are employed to describe elements and components described herein. This is done merely for convenience and to give a general sense of the scope of the invention. This description should be read to include one or at least one and the singular also includes the plural unless it is obvious that it is meant otherwise.

Group numbers corresponding to columns within the Periodic Table of the elements use the "New Notation" convention as seen in the CRC Handbook of Chemistry and Physics, 81st Edition (2000-2001).

Unless otherwise defined, all technical and scientific terms used herein have the same meaning as commonly understood by one of ordinary skill in the art to which this invention belongs. Although methods and materials similar or equivalent to those described herein can be used in the practice or testing of embodiments of the present invention, suitable methods and materials are described below. In case of conflict, the present specification, including definitions, will control. In addition, the materials, methods, and examples are illustrative only and not intended to be limiting.

In step (a) of the process of the invention, hydrogen (H₂) and CF₃CF=CHF (HFC-1225ze) are added to a reaction vessel containing a hydrogenation catalyst. HFC-1225ye may exist as two configurational isomers, E, or Z. HFC-1225ye as used herein refers to the isomers, *E-*HFC-1225ye (CAS RN 5595-10-8) or Z-HFC-1225ye (CAS RN 5528-43-8), as well as any combinations or mixtures of such isomers. HFC-1225ye may be prepared by dehydrofluorination of 1,1,1,2,3,3-hexafluoropropane (HFC-236ea) or by dehydrofluorination of 1,1,1,2,2,3-hexafluoropropane (HFC-236cb) as disclosed in U.S. Patent Application No. 11/264,183 and U.S. Patent 6,369,284.

In one embodiment, reaction vessels for step (a) include heated tubular reactors suitable for carrying out reactions in the vapor phase. A number of reactor configurations are possible, including vertical and horizontal orientation of the reactor and different modes of contacting the starting material(s) with the hydrogenation catalyst.

In step (b) of the process of the invention, the reaction of CF₃ CF=CHF with hydrogen employs a hydrogenation catalyst. In one embodiment, the hydrogenation catalyst contains a metal (e.g, a Group VIII metal or rhenium). In one embodiment, the metal is supported (e.g., Pd supported on alumina, aluminum fluoride, or carbon). In another embodiment, the metal is unsupported (e.g., Raney nickel). In one embodiment, the catalyst is a Carbon-supported metal catalyst. In another embodiment, the catalyst is Pd on carbon. In one embodiment, the carbon support is washed with acid prior to depositing the metal on it. Procedures for preparing a catalyst of Group VIII metal or rhenium on an acid-washed carbon support are disclosed in U.S. Patent No. 5,136,113.

In one embodiment, the contact of CF₃CF=CHF with hydrogen in the presence of a hydrogenation catalyst is suitably conducted at a temperature in the range of from about 50 °C. to about 300 °C. In another embodiment, the contact of CF₃CF=CHF with hydrogen in the presence of a hydrogenation catalyst is suitably conducted at a temperature in the range of from about 50 °C. to about 200 °C. In one embodiment, contact time is from about 5 to about 100 seconds. In another embodiment, contact time is from about 10 to about 30 seconds.

In one embodiment, the molar ratio of hydrogen to CF₃CF=CHF typically is in the range from 1:1 to 50:1. In another embodiment, the molar ratio of hydrogen to CF₃CF=CHF is from 1.5:1 to 25:1. In yet another embodiment, the molar ratio of hydrogen to CF₃CF=CHF is from 2:1 to 10:1. In one embodiment, hydrogen can be fed in the pure state. In another embodiment, hydrogen can be fed diluted with inert gas (e.g., nitrogen, helium or argon).

The product from the reaction of H₂ with CF₃CF=CHF is primarily CF₃CHFCH₂F. In some embodiment, by-products that may be present include CF₃CHFCH₃ (HFC-254eb).

In one embodiment, the CF₃CHFCH₂F prepared in step (b) may be separated from excess hydrogen (if any), unconverted CF3CF=CHF (if any), and reaction by-products by conventional techniques, such as distillation. The recovered HFC-245eb is then directed to step (c).

In another embodiment of the invention, the product effluent from step (b) is carried directly to step (c) without further treatment or purification.

Step (b) is typically conducted at atmospheric pressure or superatmospheric pressure. In one embodiment, atmospheric and superatmospheric pressures (e.g., pressure from about 100 kPa to 7000 kPa) are typically employed.

In step (c) of the process of the invention, CF₃CHFCH₂F produced in step (b) is dehydrofluorinated. In one embodiment, the dehydrofluorination may be carried out in the vapor phase. Vapor phase dehydrofluorination of a hydrofluorocarbon may be suitably carried out using typical dehydrofluorination catalysts. The present dehydrofluorination may be carried out using any dehydrofluorination catalyst known in the art. These catalysts include, but are not limited to, aluminum fluoride; gamma alumina, fluorided alumina; metals on aluminum fluoride; metals on fluorided alumina; oxides, fluorides, and oxyfluorides of magnesium, zinc and mixtures of magnesium and zinc and/or aluminum; lanthanum oxide and fluorided lanthanum oxide; chromium oxides, fluorided chromium oxides, and cubic chromium trifluoride; carbon, acid-washed carbon, activated carbon, three dimensional matrix carbonaceous materials; and metal compounds supported on carbon. The metal compounds are oxides, fluorides, and oxyfluorides of at least one metal selected from the group consisting of sodium, potassium, rubidium, cesium, yttrium, lanthanum, cerium, praseodymium, neodymium, samarium, chromium, iron, cobalt, rhodium, nickel, copper, zinc, and mixtures thereof.

In one embodiment, dehydrofluorination catalysts include aluminum fluoride, fluorided alumina, metals on aluminum fluoride, and metals on fluorided alumina, as disclosed in U. S. Patent No. 5,396,000. Fluorided alumina and aluminum fluoride can be prepared as described in U. S. Patent No. 4,902,838. Suitable metals include chromium, magnesium (e.g., magnesium fluoride), Group VIIB metals (e.g., manganese), Group IIIB metals (e.g., lanthanum), and zinc. In one embodiment, such metals are present as halides (e.g., fluorides), as oxides and/or as oxyhalides. Metals on aluminum fluoride and metals on fluorided alumina can be prepared by procedures as described in U.S. Patent No. 4,766,260. In one embodiment, when supported metals are used, the total metal content of the catalyst is from about 0.1 to 20 percent by weight, typically from about 0.1 to 10 percent by weight. Preferred catalysts include catalysts consisting essentially of aluminum fluoride and/or fluorided alumina.

In another embodiment, dehydrofluorination catalysts include oxides, fluorides, and oxyfluorides of magnesium, zinc and mixtures of magnesium and zinc and/or aluminum. In one embodiment, a suitable catalyst may be prepared, for example by drying magnesium oxide until essentially all water is removed, e.g., for about 18 hours at about 100°C. The dried material is then transferred to the reactor to be used. The temperature is then gradually increased to about 400°C while maintaining a flow of nitrogen through the reactor to remove any remaining traces of moisture from the magnesium oxide and the reactor. The temperature is then lowered to about 200°C and a fluoriding agent, such as HF, or other vaporizable fluorine containing compounds such as HF, SF₄, CCl₃F, CCl₂F₃, CHF₃, CHClF₂ or CCl₂FCClF₂, optionally diluted with an inert gas such as nitrogen, is passed through the reactor. The inert gas or nitrogen can be gradually reduced until only HF or other vaporizable fluorine containing compounds is being passed through the reactor. At this point, the temperature can be increased to about 450°C and held at that temperature to convert the magnesium oxide to a fluoride content corresponding to at least 40 percent by weight, e.g., for 15 to 300 minutes, depending on the fluoriding agent flow rate and the catalyst volume. The fluorides are in the form of magnesium fluoride or magnesium oxyfluoride; the remainder of the catalyst is magnesium oxide. It is understood in the art that fluoriding conditions such as time and temperature can be adjusted to provide higher than 40 percent by weight fluoride-containing material.

In another embodiment, a suitable procedure for the catalyst preparation is to add ammonium hydroxide to a solution of magnesium nitrate and, if present, zinc nitrate and/or aluminum nitrate. The ammonium hydroxide is added to the nitrate solution to a pH of about 9.0 to 9.5. At the end of the addition, the solution is filtered, the solid obtained is washed with water, dried and slowly heated to 500°C, where it is calcined. The calcined product is then treated with a suitable fluorine-containing compound as described above.

In yet another embodiment, a procedure for the preparation of metal (i.e., magnesium, optionally containing also zinc and/or aluminum) fluoride catalysts containing one or more metal fluorides is to treat an aqueous solution of the metal(s) halide(s) or nitrate(s) in deionized water with 48 percent aqueous HF with stirring. Stirring is continued overnight and the slurry evaporated to dryness on a steam bath. The dried solid is then calcined in air at 400 °C for about four hours, cooled to room temperature, crushed and sieved to provide material for use in catalyst evaluations.

In yet another embodiment, dehydrofluorination catalysts include lanthanum oxide and fluorided lanthanum oxide. Suitable fluorided lanthanum oxide compositions may be prepared in any manner analogous to those known to the art for the preparation of fluorided alumina. In one embodiment, the catalyst composition can be prepared by fluorination of lanthanum oxide.

In another embodiment suitable catalyst compositions may be prepared by precipitation of lanthanum as the hydroxide, which is thereafter dried and calcined to form an oxide, a technique well known to the art. The resulting oxide can then be pretreated as described herein.

The catalyst composition can be fluorinated to the desired fluorine content by pretreatment with a fluorine-containing compound at elevated temperatures, e.g., at about 200°C to about 450°C. The pretreatment with a vaporizable fluorine-containing compound such as HF, SF₄, CCl₃F, CCl₂F₃, CHF₃, CHClF₂ or CCl₂FCClF₂ can be done in any convenient manner including in the reactor which is to be used for carrying out the dehydrofluorination reaction. By vaporizable fluorine-containing compound is meant a fluorine containing compound which, when passed over the catalyst at the indicated conditions, will fluorinate the catalyst to the desired degree.

In another embodiment, a suitable catalyst may be prepared, for example, by drying La₂O₃ until essentially all moisture is removed, e.g., for about 18 hours at about 400°C. The dried catalyst is then transferred to the reactor to be used. The temperature is then gradually increased to about 400°C while maintaining a flow of N₂ through the reactor to remove any remaining traces of moisture from the catalyst and the reactor. The temperature is then lowered to about 200°C and the vaporizable fluorine-containing compound is passed through the reactor. In some embodiments, nitrogen or other inert gases can be used as diluents. The N₂ or other inert diluents can be gradually reduced until only the vaporizable fluorine-containing compound is being passed through the reactor. At this point the temperature can be increased to about 450°C and held at that temperature to convert the La₂O₃ to a fluorine content corresponding to at least 80 percent LaF₃ by weight, e.g., for 15 to 300 minutes, depending on the flow of the fluorine containing compound and the catalyst volume.

In yet another embodiment, the catalyst is prepared by adding ammonium hydroxide to a solution of La(NO₃)₃·6H2O. The ammonium hydroxide is added to the nitrate solution to a pH of about 9.0 to 9.5. At the end of the addition, the solution is filtered, the solid obtained is washed with water, and slowly heated to about 400 °C, where it is calcined. The calcined product is then treated with a suitable vaporizable fluorine-containing compound as described above.

In other embodiments, dehydrofluorination catalysts include chromium oxides, fluorided chromium oxides, cubic chromium trifluoride, and cobalt substituted chromium oxide. Cobalt-substituted chromium oxide is disclosed in US Patent 7,217,678. Cubic chromium trifluoride may be prepared from CrF₃XH₂O, where X is 3 to 9, preferably 4, by heating in air or an inert atmosphere (e.g., nitrogen or argon) at a temperature of about 350 °C to about 400 °C for 3 to 12 hours, preferably 3 to 6 hours.

In some embodiments, cubic chromium trifluoride is useful by itself. In other embodiments cubic chromium trifluoride is used together with other chromium compounds, as a dehydrofluorination catalyst. Preparation of cubic chromium trifluoride is described in U. S. Patent No. 6,031,141. In one embodiment, the catalyst compositions comprising chromium have at least 10 weight percent of the chromium in the form of cubic chromium trifluoride. In another embodiment, the catalyst compositions comprising chromium have at least 25 percent of the chromium in the form of cubic chromium trifluoride. In yet another embodiment, the catalyst compositions comprising chromium have at least 60 percent of the chromium in the form of cubic chromium trifluoride. In some embodiments, the chromium, including the cubic chromium trifluoride is supported on and/or physically mixed with materials such as carbon, aluminum fluoride, fluorided alumina, lanthanum fluoride, magnesium fluoride, calcium fluoride, zinc fluoride and the like. In yet another embodiment, combinations including cubic chromium trifluoride in combination with magnesium fluoride and/or zinc fluoride are employed.

In yet another embodiment, dehydrofluorination catalysts include activated carbon, or three dimensional matrix carbonaceous materials as disclosed in U. S. Patent No. 6,369,284, or carbon or metals such as sodium, potassium, rubidium, cesium, yttrium, lanthanum, cerium, praseodymium, neodymium, samarium, chromium, iron, cobalt, rhodium, nickel, copper, zinc, and mixtures thereof, supported on carbon as disclosed in U. S. Patent No. 5,268,122. In one embodiment, carbon from any of the following sources are useful as catalysts; wood, peat, coal, coconut shells, bones, lignite, petroleum-based residues and sugar. Commercially available carbons which may be used include those sold under the following trademarks: Barneby & Sutcliffe™, Darco™, Nucharm, Columbia JXN™, Columbia LCK™, Calgon PCB, Calgon BPL™, Westvaco™, Norit™, and Barnaby Cheny NB™

In one embodiment, carbon includes acid-washed carbon (e.g., carbon which has been treated with hydrochloric acid or hydrochloric acid followed by hydrofluoric acid). Acid treatment is typically sufficient to provide carbon that contains less than 1000 ppm of ash. Suitable acid treatment of carbon is described in U.S. Patent No. 5,136,113. In another embodiment, the carbon also includes three dimensional matrix porous carbonaceous materials. Examples are those described in U.S. Patent No. 4,978,649. Of note are three dimensional matrix carbonaceous materials which are obtained by introducing gaseous or vaporous carboncontaining compounds (e.g., hydrocarbons) into a mass of granules of a carbonaceous material (e.g., carbon black); decomposing the carboncontaining compounds to deposit carbon on the surface of the granules; and treating the resulting material with an activator gas comprising steam to provide a porous carbonaceous material. A carbon-carbon composite material is thus formed.

In some embodiments, the physical shape of the catalyst may, for example, include pellets, powders or granules. In other embodiments, for catalysts supported on carbon, the carbon may be in the form of powder, granules, or pellets, or the like. In some embodiments, catalysts that have not been fluorided may be treated with HF before use. It is thought that this converts some of the surface oxides to oxyfluorides. This pretreatment can be accomplished by placing the catalyst in a suitable container (which can be the reactor to be used to perform the reaction of the instant invention) and thereafter, passing HF over the dried catalyst so as to partially saturate the catalyst with HF. This is conveniently carried out by passing HF over the catalyst for a period of time (e.g., about 15 to 300 minutes) at a temperature of, for example, about 200 °C to about 450 °C.

In one embodiment, the catalytic dehydrofluorination may be suitably conducted at a temperature in the range of from about 200 °C to about 500 °C. In another embodiment, the catalytic dehydrofluorination may be suitably conducted at a temperature in the range of from about 300 °C to about 450 °C. In one embodiment, the contact time is from about 1 to about 450 seconds. In another embodiment, the contact time is from about 10 to about 120 seconds.

The reaction pressure can be subatmospheric, atmospheric or superatmospheric. In one embodiment, near atmospheric pressures are utilized. In another embodiment, the dehydrofluorination can be run under reduced pressure (i.e., pressures less than one atmosphere).

In one embodiment, the catalytic dehydrofluorination can be carried out in the presence of an inert gas such as nitrogen, helium, or argon. The addition of an inert gas can be used to increase the extent of dehydrofluorination. In some embodiments; the mole ratio of inert gas to hydrofluorocarbon undergoing dehydrofluorination is from about 5:1 to about 1:1. In one embodiment, nitrogen is the preferred inert gas.

In another embodiment, the catalytic dehydrofluorination is carried out in the presence of hydrogen.

In another embodiment, the dehydrofluorination of CF₃CHFCH₂F is accomplished using a basic aqueous solution in the presence of a non-aqueous, non-alcoholic solvent in which the CF₃CHFCH₂F is at least partially miscible. In one embodiment, the base in the basic aqueous solution includes alkali metal or alkaline earth metal hydroxides and oxides, or mixtures thereof, which can include without limitation lithium hydroxide, sodium hydroxide, potassium hydroxide, calcium hydroxide, magnesium oxide, calcium oxide, sodium carbonate, potassium carbonate, sodium phosphate, potassium phosphate, or mixtures thereof.

The amount of base (in the basic aqueous solution) required to convert CF₃CHFCH₂F to a hydrofluoroolefin is approximately the stoichiometric quantity or about 1 mole of base to one mole of CF₃CHFCH₂F. In one embodiment, it may desirable (e.g., to increase reaction rate) to employ a ratio of base to CF₃CHFCH₂F of greater than one. In some embodiments, large excesses of base (in the basic aqueous solution) are to be avoided as further reaction of the desired hydrofluoroolefin may occur. Thus, in some embodiments, it may be necessary to employ an amount of base (in the basic aqueous solution) that is slightly below the stoichiometric amount so as to minimize secondary reactions. Thus, in one embodiment, the molar ratio of base (in the basic aqueous solution) to CF₃CHFCH₂F is from about 0.75:1 to about 10:1. In another embodiment, the molar ratio of base (in the basic aqueous solution) to CF₃CHFCH₂F is from about 0.9:1 to about 5:1. In yet another embodiment, the molar ratio of base to CF₃CHFCH₂F is from about 1:1 to about 4:1.

In one embodiment, the dehydrofluorination is conducted within a temperature range at which the CF₃CHFCH₂F will dehydrofluorinate. In one embodiment, such temperatures can be from about 20 °C to about 150 °C. In another embodiment, the reaction is conducted in the range of from about 30 °C to about 110 °C. In yet another embodiment, the reaction is carried out in the range of from about 40 °C to about 90 °C. The reaction pressure is not critical. The reaction can be conducted at atmospheric pressure, super-atmospheric pressure, or under reduced pressure. In one embodiment, the reaction is carried out at atmospheric pressure.

In one embodiment, a solid base (e.g., KOH, NaOH, LiOH or mixtures thereof) is dissolved in water, or alternatively, a concentrated solution of a base (e.g., 50% by weight aqueous potassium hydroxide) is diluted to the desired concentration with water. The non-aqueous, non-alcoholic solvent for the method is then added with agitation under otherwise ambient conditions. In one embodiment, a solvent for the reaction can be a nitrile, ether, amide, ketone, sulfoxide, phosphate ester, or mixtures thereof. In another embodiment, the solvent is selected from the group consisting of acetonitrile, propionitrile, butyronitrile, methyl glutaronitrile, adiponitrile, benzonitrile, ethylene carbonate, propylene carbonate, methyl ethyl ketone, methyl isoamyl ketone, diisobutyl ketone, anisole, 2-methyltetrahydrofuran, tetrahydrofuran, dioxane, diglyme, triglyme, tetraglyme, N,N-dimethyl formamide, N,N-dimethyl acetamide, N-methyl pyrrolidinone, sulfolane, dimethyl sulfoxide, perfluoro-N-methyl morpholine, perfluorotetrehydrofuran, and mixtures thereof. Preferred solvents include acetonitrile, adiponitrile, 2-methyl tetrahydrofuran, tetrahydrofuran, dioxane, diglyme, and tetraglyme.

In one embodiment, the base need not be highly soluble in the solvent. An amount of a phase transfer catalyst is added to the solvent for the reaction in quantities that improve the solubility of the base therein. In one embodiment, the amount of phase transfer catalyst used will be from about 0.001 to about 10 mole percent based on the total amount of base present. In another embodiment, the amount of phase transfer catalyst used will be from about 0.01 to about 5 mole percent based on the total amount of base present. In yet another embodiment, the amount of phase transfer catalyst used will be from about 0.05 to about 5 mole percent based on the total amount of base present. In one embodiment of the invention, an aqueous or inorganic phase is present as a consequence of the base and an organic phase is present as a result of the CF₃CHFCH₂F and the non-aqueous, non-alcoholic solvent.

The phase transfer catalyst is selected from the group consisting of crown ethers, onium salts, cryptands and polyalkylene glycols and mixtures and derivatives thereof.

Crown ethers are cyclic molecules in which ether groups are connected by dimethylene linkages; the compounds form a molecular structure that is believed to be capable of "receiving" or holding the alkali metal ion of the hydroxide and to thereby facilitate the reaction. In some embodiments, it is preferred to match certain crown ether phase transfer catalysts with certain bases used in the basic aqueous solutions. In one embodiment, crown ethers include 18-crown-6, is used in combination with potassium hydroxide basic aqueous solution; 15-crown-5, is used in combination with sodium hydroxide basic aqueous solution; 12-crown-4, is used in combination with lithium hydroxide basic aqueous solution. Derivatives of the above crown ethers are also useful, e.g., dibenzo-18-crown-6, dicyclohexano-18-crown-6, and dibenzo-24-crown-8 as well as 12-crown-4. Other polyethers particularly useful in combination with basic aqueous solution made from alkali metal compounds, and especially for lithium, are described in U.S. Patent No. 4,560,759. Other compounds analogous to the crown ethers and useful for the same purpose are compounds which differ by the replacement of one or more of the oxygen atoms by other kinds of donor atoms, particularly N or S, such as hexamethyl-[14]-4,11-dieneN₄.

In some embodiments, onium salts include quaternary phosphonium salts and quaternary ammonium salts that may be used as the phase transfer catalyst in the process of the present invention; such compounds can be represented by the following formulas II and III:

R¹ R² R³ R⁴ P⁽⁺⁾ X'⁽⁻⁾ (II)

R¹ R² R³ R⁴ N⁽⁺⁾ X'⁽⁻⁾ (III)

wherein each of R¹, R², R³ and R⁴, which may be the same or different, is an alkyl group, an aryl group or an aralkyl group, and X' is selected from the group consisting of F, Cl, Br, I, OH, CO₃, HCO₃, SO₄, HSO₄, H₂PO₄, HPO₄ and PO₄. Specific examples of these compounds include tetramethylammonium chloride, tetramethylammonium bromide, benzyltriethylammonium chloride, methyltrioctylammonium chloride, tetra-n-butylammonium chloride, tetra-n-butylammonium bromide, tetra-n-butylammonium hydrogen sulfate, tetra-n-butylphosphonium chloride, tetraphenylphosphonium bromide, tetraphenylphosphonium chloride, triphenylmethylphosphonium bromide and triphenylmethylphosphonium chloride. In one embodiment, benzyltriethylammonium chloride is used under strongly basic conditions. Other useful compounds within this class of compounds include those exhibiting high temperature stabilities (e.g., up to about 200 °C.) including 4-dialkylaminopyridinium salts, tetraphenylarsonium chloride, bis(tris(dimethylamino)phosphine]iminium chloride, and tetratris[tris(dimethylamino)phosphinimino]phosphonium chloride; the latter two compounds are also reported to be stable in the presence of hot, concentrated sodium hydroxide and, therefore, can be particularly useful.

In some embodiments, polyalkylene glycol ethers are useful as phase transfer catalysts. In some embodiments, the polyalkylene glycol ethers can be represented by the formula:

R⁶O (R⁵O)ₜ R⁷ (IV)

wherein R⁵ is an alkylene group containing two or more carbon atoms, each of R⁶ and R⁷, which may be the same or different, is a hydrogen atom, an alkyl group, an aryl group or, an aralkyl group, and t is an integer of at least 2. Such compounds include, for example glycols such as diethylene glycol, triethylene glycol, tetraethylene glycol, pentaethylene glycol, hexaethylene glycol, diisopropylene glycol, dipropylene glycol, tripropylene glycol, tetrapropylene glycol and tetramethylene glycol, and monoalkyl ethers such as monomethyl, monoethyl, monopropyl and monobutyl ethers of such glycols, dialkyl ethers such as tetraethylene glycol dimethyl ether and pentaethylene glycol dimethyl ether, phenyl ethers, benzyl ethers, and polyalkylene glycols such as polyethylene glycol (average molecular weight about 300) dimethyl ether, polyethylene glycol (average molecular weight about 300) dibutyl ether, and polyethylene glycol (average molecular weight about 400) dimethyl ether, and ethoxylated furfurylalcohol.Among them, compounds wherein both R-⁶ and R-⁷ are alkyl groups, aryl groups or aralkyl groups are preferred.

In other embodiments, cryptands are another class of compounds useful in the present as phase transfer catalysts. These are three-dimensional polymacrocyclic chelating agents that are formed by joining bridgehead structures with chains that contain properly spaced donor atoms. For example, bicyclic molecules that result from joining nitrogen bridgeheads with chains of (--OCH₂CH₂--) groups as in 2.2.2-cryptand (4,7,13,16,21,24-hexaoxa-1,10-diazabicyclo-(8.8.8)hexacosane; available under the brand names Cryptand^{™} 222 and Kryptofix^{™} 222). The donor atoms of the bridges may all be O, N, or S, or the compounds may be mixed donor macrocycles in which the bridge strands contain combinations of such donor atoms.

Combinations and mixtures of the above described phase transfer catalysts from within one of the groups may also be useful as well as combinations or mixtures two or more phase transfer catalysts selected from more than one group, for example, crown ethers and oniums, or from more than two of the groups, e.g., quaternary phosphonium salts and quaternary ammonium salts, and crown ethers and polyalkylene glycol ethers.

The dehydrofluorination reactions of this invention may be carried out in either a batch or a continuous mode. In some embodiments, the dehydrofluorination process is carried out in batch mode and in other embodiments, the dehydrofluorination continuous mode. In one embodiment, in the batch mode, the above described components are combined in a suitable vessel for a time sufficient to convert at least a portion of the CF₃CHFCH₂F to CF₃CF=CH₂ and then the CF₃CF=CH₂ is recovered from the reaction mixture.

In another embodiment, in a continuous mode of operation, the reaction vessel is charged with the basic aqueous solution, non-aqueous, non-alcoholic solvent, and phase transfer catalyst; the CF₃CHFCH₂F is then fed to the reactor. The reaction vessel is fitted with a condenser cooled to a temperature sufficient to reflux the CF₃CHFCH₂F, but permit the CF₃CF=CH₂ to exit the reaction vessel and collect in an appropriate vessel such as cold trap.

Products formed in step (c) comprise HF, CF₃CF=CH₂, and, if CF₃CHFCH₃ is present in the feed mixture, CF₃CH=CH₂. In one embodiment, the HFC-1234yf is typically separated from the lower boiling products and higher boiling products by conventional means (e.g., distillation).

The reactor, distillation columns, and their associated feed lines, effluent lines, and associated units used in applying the processes of this invention should be constructed of materials resistant to hydrogen fluoride and hydrogen chloride. Typical materials of construction, well-known to the fluorination art, include stainless steels, in particular of the austenitic type, the well-known high nickel alloys, such as Monel^{™} nickel-copper alloys, Hastelloy^{™} nickel-based alloys and, Inconel^{™} nickel-chromium alloys, and copper-clad steel.

### Legend

| | |
|---|---|
| HFC-245eb is CF₃CHFCH₂F | HFC-254eb is CF₃CHFCH₃ |
| HFC-1225ye is E- or Z-CF₃CF=CHF | HFC-1234yf is CF₃CF=CH₂ |
| HFC-1234ze is E- or Z-CF₃CH=CHF | |

### EXAMPLES

### Example 1

Example 1 demonstrates the hydrogenation of HFC-1225ye. An inconel tube (5/8 inch (1.59 cm) OD) was filled with 16 cc (14.45 gm) of 0.5% palladium on acid washed carbon (6x10 mesh). The catalyst was heated to 400° for 7 minutes under a nitrogen purge of 20 sccm (3.33 x 10⁻⁷ m³/s) and then lowered to 100° for 13 minutes. The temperature was raised to 200°C for 45 minutes under a nitrogen purge of 40 sccm (6.67 x 10⁻⁷ m³/s). The flow of nitrogen was reduced to 20 sccm (3.33 x 10⁻⁷ m³) and hydrogen introduced at 10 sccm (1.67 x 10⁻⁷ m³/s) for 60 minutes. While maintaining the same nitrogen flow, hydrogen was increased to 20 sccm (3.33 x 10⁻⁷ m³/s) for 30 minutes. While maintaining the flow of hydrogen, nitrogen was reduced to 10 sccm (1.67 x 10⁻⁷ m³/s) for 60 minutes. The nitrogen was shut off and the hydrogen was increased to 40 sccm (6.67 x 10⁻⁷ m³/s) for 130 minutes.

The temperature of the reactor was lowered to 85°C and HFC-1225ye was fed at 61 sccm (1.02 x 10⁻⁶ m³/s) and hydrogen at 85 sccm (1.42 x 10⁻⁶ m³/s). The effluent of the reactor was analyzed by GCMS to contain 92% HFC-245eb and 8% HFC-254eb .

### Example 2

Example 2 demonstrates that the dehydrofluorination of HFC-245eb

A three neck 2 L flask was equipped with a water ice condenser, thermocouple, and over-head stirrer. The effluent of the condenser was passed through a CaSO₄ drier and then through activated molecular sieves and a stainless steel trap with dip tube immersed in dry ice /acetone. A Krytox™ oil bubbler at the exit of the stainless steel trap prevented contamination of the trapped product by moisture.

The flask was charged with water (736 ml), THF (200 ml), KOH pellets (180 g, 3.21 mol), and Aliquat™ 336 (3.13 gm, 7.74 x 10⁻³ mol). While vigorously stirring, HFC-245eb was added at a rate of 100 sccm. About 247 gm of crude product was collected containing 96.1% HFC-1234yf, 0.5% HFC-Z-1234ze, 0.1% HFC-E-1234ze and 2.9% unreacted HFC-245eb. Very little exotherm was observed while feeding the HFC-245eb.

### Example 3

Example 3 illustrates the conversion of HFC-1225ye into HFC-1234yf in a single sequence.

An inconel tube (5/8 inch (1.59 cm) OD) is filled with 16 cc (14.45 gm) of 0.5% palladium on acid washed carbon (6x10 mesh). The catalyst is activated as described in Example 1. The temperature of the reactor is adjusted to 85°C and HFC-1225ye is fed at 61 sccm (1.02 x 10⁻⁶ m³/s) and hydrogen at 85 sccm (1.42 x 10⁻⁶ m³/s). The effluent of the reactor is fed directly into a second catalyst bed.

This second catalyst bed consists of gamma alumina (8.44 gm, 13 ml) ground to 12/20 mesh (0.84 to 1.68 mm). The gamma alumina catalyst is activated by heating at 175°C for 30 minutes under a nitrogen purge (25 sccm, 4.2x10⁻⁷ m³/s). HF is fed at 25 sccm (4.2x10⁻⁷ m³/s) for 85 minutes. The temperature is then raised to 250°C, the HF flow increased to 40 sccm (6.7x10⁻⁷ m³/s), and the nitrogen decreased to 10 sccm (1.7x10⁻⁷ m³/s) for 15 hours. The temperature is then raised to 350°C while maintaining flows for 150 minutes, and then the temperature was raised to 450°C while maintaining flows for 230 minutes. When the temperature of the second fluorided alumina catalyst bed is at 400°C, the HFC-245eb exiting the Pd/C catalyst bed reacts to form the desired HFC-1234yf and HF in high conversion.

### Example 4

Example 4 demonstrates the synthesis of HFC-1234yf from HFC-245eb by dehydrofluorination over Fluorided Alumina catalyst

A Hastelloy™ tube reactor (2.54 cm OD X 2.17 cm ID X 24.1 cm L) was filled with 25 cc of gamma-alumina ground to 12-20 mesh (0.84 to 1.68 mm). The catalyst was activated by heating at 200°C for 15 minutes under a nitrogen purge and then reacted with a HF/N₂ mixture heated up to 425°C to yield 16.7 gm of activated fluorided alumina.

At a temperature of 350°C, 10 sccm of nitrogen (1.7 x 10⁻⁷ m³/s) and 15 sccm (2.5 x 10⁻⁷ m³/s) of HFC-245eb were mixed and flowed through the reactor. The temperature was then raised to 400°C, the flow rates held constant. The effluent from the reactor at both temperatures was sampled and analyzed by GC and ¹⁹F NMR (see Table 1).

**TABLE 1**

| Temp., °C | N₂ flow (sccm) | 245eb flow (sccm) | Concentrations, (Mole %) | | |
|---|---|---|---|---|---|
| | | | 1234yf | 245eb | Unks |
| 350 | 10 | 15 | 84.2 | 12.8 | 3.0 |
| 400 | 10 | 15 | 91.3 | 1.9 | 6.8 |

| | | | | | |
|---|---|---|---|---|---|
| Unks = unknowns | | | | | |

### Example 5

Example 5 demonstrates the synthesis of HFC-1234yf by dehydrofluorination of HFC-245eb over a Carbon Catalyst

A Hastelloy™ nickel alloy reactor (2.54 cm OD X 2.17 cm ID X 24.1 cm L) was charged with 14.32 g (25 mL) of spherical (8 mesh) three dimensional matrix porous carbonaceous material prepared substantially as described in U.S. Patent No. 4,978,649. The packed portion of the reactor was heated by a 5" X 1" ceramic band heater clamped to the outside of the reactor. A thermocouple positioned between the reactor wall and the heater measured the reactor temperature. After charging the reactor with the carbonaceous material, nitrogen (10 ml/min, 1.7X10⁻⁷ m³/s) was passed through the reactor and the temperature was raised to 200 °C during a period of one hour and maintained at this temperature for an additional 4 hours. The reactor temperature was then raised to the desired operating temperature and a flow of CF₃CHFCH₂F and nitrogen was started through the reactor.

At a temperature of 400 °C, a mixture of 10 sccm (1.7 x 10⁻⁷ m³/s) of nitrogen and 15 sccm (2.5 x 10⁻⁷ m³/s) of HFC-245eb were passed through the reactor giving a contact time of 60 seconds. The flows were reduced to 5 sccm 8.3 x 10⁻⁸ m³/s) of nitrogen (and 7.5 sccm (1.3 x 10⁻⁷ m³/s) of HFC-245eb giving a contact time of 120 seconds. The effluent from the reactor was sampled under both sets of conditions was analyzed by GC and ¹⁹F NMR (see Table 2).

**TABLE 2**

| Temp., °C | N₂ flow (sccm) | 245eb flow (sccm) | Concentrations, Mole% | | |
|---|---|---|---|---|---|
| | | | 1234yf | 245eb | Unks |
| 400 | 10 | 15 | 6.0 | 93.9 | 0.1 |
| 400 | 5 | 7.5 | 22.8 | 76.4 | 0.8 |

| | | | | | |
|---|---|---|---|---|---|
| Unks = unknowns | | | | | |

### Example 6

Example 6 demonstrates dehydrofluorination of HFC-245eb over Cr/Co catalyst.

Cobalt-substituted chromium oxide (Cr/Co 95/5, 10 cc, 12-20 mesh (1.68-0.84 mm)), prepared as described in U. S. Patent No. 7,217,678, was placed in a 30.5 cm x 1.27 cm o.d. Hastelloy® tube. The tube was connected to a reactor system and surrounded with a electrically-heated furnace. The catalyst was activated by first drying at 125°C for one hour under a nitrogen purge (38 sccm, 6.3x10⁻⁷ m³/s) followed by one hour at 175°C. Hydrogen fluoride gas (19 sccm, 3.2x10⁻⁷ m3/s) was then added to the nitrogen flow. After one hour the flow of HF was increased to 38 sccm (6.3x10⁻⁷ m³/s). After another hour, the HF flow was increased to 60 sccm (1.0x10⁻⁶ m³/s) and the nitrogen decreased to 15 sccm (8.3x10⁻⁸ m³/s). After one hour, the temperature of the reactor was increased to 250°C, held for two hours, and then increased to 400°C and held for an additional two hours. The flow of HF was then stopped and the catalyst purged with nitrogen (20 sccm, (3.3x10⁻⁷ m³/s) as the temperature was reduced to 275°C.

A mixture of CF₃CHFCH₂F (HFC-245eb) and nitrogen (1:2 molar ratio) was fed to the above catalyst at 250°C; the contact time was nominally 30 seconds. The analysis of the reactor effluent as determined by GC-MS is given in Table 3.

**Table 3**

| Example | % 245cb | % 1234yf | % 1234 | %245eb |
|---|---|---|---|---|
| 6 | 0.6 | 53.9 | 0.7 | 44.8 |
| 7 | 0.6 | 52.5 | 0.7 | 46.2 |

### Example 7

Example 7 demonstrates dehydrofluorination of HFC-245eb over Cr/Co catalyst in the presence of hydrogen.

A mixture of CF₃CHFCH₂F (HFC-245eb) and hydrogen (1:2 molar ratio) was fed to the catalyst and reactor described in Example 6 at 250°C. The contact time was nominally 30 seconds. The analysis of the reactor effluent as determined by GC-MS is given in Table 3.

Note that not all of the activities described above in the general description or the examples are required, that a portion of a specific activity may not be required, and that one or more further activities may be performed in addition to those described. Still further, the order in which activities are listed are not necessarily the order in which they are performed.

In the foregoing specification, the concepts have been described with reference to specific embodiments. However, one of ordinary skill in the art appreciates that various modifications and changes can be made without departing from the scope of the invention as set forth in the claims below. Accordingly, the specification and figures are to be regarded in an illustrative rather than a restrictive sense, and all such modifications are intended to be included within the scope of invention.

Benefits, other advantages, and solutions to problems have been described above with regard to specific embodiments. However, the benefits, advantages, solutions to problems, and any feature(s) that may cause any benefit, advantage, or solution to occur or become more pronounced are not to be construed as a critical, required, or essential feature of any or all the claims.

It is to be appreciated that certain features are, for clarity, described herein in the context of separate embodiments, may also be provided in combination in a single embodiment. Conversely, various features that are, for brevity, described in the context of a single embodiment, may also be provided separately or in any subcombination. Further, reference to values stated in ranges includes each and every value within that range.

## Claims

1. A process for producing CF₃CF=CH₂ Comprising the steps of:
(a) adding hydrogen and CF₃CF=CHF to a reaction vessel containing a hydrogenation catalyst;
(b) reacting said CF₃CF=CNF with hydrogen over said hydrogenation catalyst to produce CF₃CHFCH₂F; and
(c) dehydrohalogenating CF₃CHFCH₂F in the vapor phase over a catalyst selected from the group consisting of aluminum fluoride; gamma alumina, fluorided alumina; metals on aluminum fluoride; metals on fluorided alumina; oxides, fluorides, and oxyfluorides of magnesium, zinc and mixtures of magnesium and zinc and/or aluminum; lanthanum oxide and fluorided lanthanum oxide; chromium oxides, fluorided chromium oxides, cobalt-substituted chromium oxides, and cubic chromium trifluoride; carbon, acid-washed carbon, activated carbon, three dimensional matrix carbonaceous materials; and metal compounds supported on carbon, to produce CF₃CF=CH₂.

2. The process of claim 1 wherein the ratio of hydrogen: CF₃CF=CHF is from 1:1 to 50:1.

3. The process of claim 1 wherein the dehydrohalogenation of CF₃CHFCH₂F occurs in the presence of hydrogen.

4. The process of claim 1 wherein the metal compounds are oxides, fluorides, and oxyfluorides of at least one metal selected from the group consisting of sodium, potassium, rubidium, cesium, yttrium, lanthanum, cerium, praseodymium, neodymium, samarium, chromium, iron, cobalt, rhodium, nickel, copper, zinc, and mixtures thereof.

5. The process of claim 1 wherein the hydrogenation catalyst is a carbon-supported metal catalyst.

6. The process of claim 5 wherein the carbon-supported metal is a Group VIII metal or rhenium.

7. The process of claim 5 wherein the carbon-supported metal is a carbon-supported palladium catalyst.

8. The process of claim 5 wherein the dehydrofluorination catalyst consists essentially of gamma alumina, aluminum fluoride, fluorided alumina, and mixtures thereof.

9. The process of claim 5 wherein the dehydrofluorination catalyst consists essentially of chromium oxide,

10. A process for producing CF₃CF=CH₂ comprising the steps of:
(a) adding hydrogen and CF₃CF=CHF to a reaction vessel containing a hydrogenation catalyst;
(b) reacting said CF₃CF=CHF with hydrogen over said hydrogenation catalyst to produce CF₃CHFCH₂F; and
(c) dehydrohalogenating CF₃CHFCH₂F by reacting with a basic aqueous solution in the presence of a non-aqueous, non- alcoholic solvent, and in the presence of a phase transfer catalyst selected from the group consisting of crown ethers, onium salts, cryptands, polyalkylene glycols, and mixtures thereof.

11. The process of claim 10 wherein the basic aqueous solution is made from a base selected from the group consisting of lithium hydroxide, sodium hydroxide, potassium hydroxide, calcium hydroxide, magnesium oxide, calcium oxide, sodium carbonate, potassium carbonate, sodium phosphate, potassium phosphate, and mixtures thereof.

12. The process of claim 10 wherein the solvent is selected from the group consisting of alkyl and aryl nitriles, alkyl and aryl ethers, amides, ketones, sulfoxides, phosphate esters and mixtures thereof.

13. The process of claim 12 wherein the phase transfer catalyst is either 18-crown-6, 15-crown-5, a quaternary ammonium salt or mixtures thereof.

14. The process of claim 13 wherein the base In the basic aqueous solution is potassium hydroxide and the phase transfer catalyst is either 18-crown-6, methyltrioctylammonium chloride or mixtures thereof.

## Patentansprüche

1. Verfahren zur Herstellung von CF₃CF=CH₂, umfassend die Schritte des:
(a) Eingebens von Wasserstoff und CF₃CF=CHF in ein Reaktionsgefäß, das einen Hydrierungskatalysator enthält;
(b) Reagierens des CF₃CF=CHF mit Wasserstoff über dem Hydrierungskatalysator, um CF₃CHFCH₂F herzustellen; und
(c) Dehydrohalogenierens des CF₃CHFCH₂F in der Dampfphase über einem Katalysator ausgewählt aus der Gruppe bestehend aus Aluminiumfluorid; gamma-Aluminiumoxid, fluoridiertem Aluminiumoxid; Metallen auf Aluminiumfluorid; Metallen auf fluoridiertem Aluminiumoxid; Oxiden, Fluoriden und Oxyfluoriden von Magnesium, Zink und Mischungen von Magnesium und Zink und/oder Aluminium; Lanthanoxid und fluoridiertem Lanthanoxid; Chromoxiden, fluoridierten Chromoxiden, kobaltsubstituierten Chromoxiden und kubischem Chromtrifluorid; Kohlenstoff, säuregewaschenem Kohlenstoff, Aktivkohle, dreidimensionalen kohlenstoffhaltigen Matrixmaterialien; und Metallverbindungen, die auf Kohlenstoff geträgert sind, um CF₃CF=CH₂ herzustellen.

2. Verfahren nach Anspruch 1, wobei das Verhältnis von Wasserstoff : CF₃CF=CHF 1:1 bis 50:1 beträgt.

3. Verfahren nach Anspruch 1, wobei die Dehydrohalogenierung von CF₃CHFCH₂F in Gegenwart von Wasserstoff erfolgt.

4. Verfahren nach Anspruch 1, wobei die Metallverbindungen Oxide, Fluoride und Oxyfluoride von mindestens einem Metall sind ausgewählt aus der Gruppe bestehend aus Natrium, Kalium, Rubidium, Cäsium, Yttrium, Lanthan, Cer, Praseodym, Neodym, Samarium, Chrom, Eisen, Kobalt, Rhodium, Nickel, Kupfer, Zink und Mischungen davon.

5. Verfahren nach Anspruch 1, wobei der Hydrierungskatalysator ein kohlenstoffgeträgerter Metallkatalysator ist.

6. Verfahren nach Anspruch 5, wobei das kohlenstoffgeträgerte Metall ein Metall der Gruppe VIII oder Rhenium ist.

7. Verfahren nach Anspruch 5, wobei das kohlenstoffgeträgerte Metall ein kohlenstoffgeträgerter Palladiumkatalysator ist.

8. Verfahren nach Anspruch 5, wobei der Dehydrofluorierungskatalysator im Wesentlichen aus gamma-Aluminiumoxid, Aluminiumfluorid, fluoridiertem Aluminiumoxid und Mischungen davon besteht.

9. Verfahren nach Anspruch 5, wobei der Dehydrofluorierungskatalysator im Wesentlichen aus Chromoxid besteht.

10. Verfahren zur Herstellung von CF₃CF=CH₂, umfassend die Schritte des:
(a) Eingebens von Wasserstoff und CF₃CF=CHF in ein Reaktionsgefäß, das einen Hydrierungskatalysator enthält;
(b) Reagierens des CF₃CF=CHF mit Wasserstoff über dem Hydrierungskatalysator, um CF₃CHFCH₂F herzustellen; und
(c) Dehydrohalogenierens des CF₃CHFCH₂F durch Reagieren mit einer basischen wässrigen Lösung in Gegenwart eines nichtwässrigen, nichtalkoholischen Lösungsmittels und in Gegenwart eines Phasenübertragungskatalysators ausgewählt aus der Gruppe bestehend aus Kronenethern, Oniumsalzen, Cryptanden, Polyalkylenglykolen und Mischungen davon.

11. Verfahren nach Anspruch 10, wobei die basische wässrige Lösung aus einer Base hergestellt ist ausgewählt aus der Gruppe bestehend aus Lithiumhydroxid, Natriumhydroxid, Kaliumhydroxid, Calciumhydroxid, Magnesiumoxid, Calciumoxid, Natriumcarbonat, Kaliumcarbonat, Natriumphosphat, Kaliumphosphat und Mischungen davon.

12. Verfahren nach Anspruch 10, wobei das Lösungsmittel aus der Gruppe ausgewählt ist bestehend aus Alkyl- und Arylnitrilen, Alkyl- und Arylethern, Amiden, Ketonen, Sulfoxiden, Phosphatestern und Mischungen davon.

13. Verfahren nach Anspruch 12, wobei der Phasenübertragungskatalysator entweder 18-Krone-6, 15-Krone-5, ein quartäres Ammoniumsalz oder Mischungen davon ist.

14. Verfahren nach Anspruch 13, wobei die Base in der basischen wässrigen Lösung Kaliumhydroxid ist und der Phasenübertragungskatalysator entweder 18-Krone-6, Methyltrioctylammoniumchlorid oder Mischungen davon ist.

## Revendications

1. Procédé de production de CF₃CF=CH₂ comprenant les étapes de:
(a) addition d'hydrogène et de CF₃CF=CHF à un récipient réactionnel contenant un catalyseur d'hydrogénation;
(b) réaction dudit CF₃CF=CHF avec de l'hydrogène sur ledit catalyseur d'hydrogénation pour produire du CF₃CHFCH₂F; et
(c) déshydrohalogénation de CF₃CHFCH₂F en phase vapeur sur un catalyseur sélectionné parmi le groupe constitué du fluorure d'aluminium; de l'alumine gamma, de l'alumine fluorée; des métaux sur du fluorure d'aluminium; des métaux sur de l'alumine fluorée; des oxydes, des fluorures, et des oxyfluorures de magnésium, du zinc et des mélanges de magnésium et de zinc et/ou d'aluminium; de l'oxyde de lanthane et de l'oxyde de lanthane fluoré; des oxydes de chrome, des oxydes de chrome fluorés, des oxydes de chrome substitués par du cobalt, et du trifluorure de chrome cubique; du charbon, du charbon lavé à l'acide, du charbon actif, des matériaux carbonés à matrice à trois dimensions; et des composés métalliques supportés sur du carbone, pour produire du CF₃CF=CH₂.

2. Procédé selon la revendication 1, dans lequel le rapport de l'hydrogène: CF₃CF=CHF est de 1:1 à 50:1.

3. Procédé selon la revendication 1, dans lequel la déshydrohalogénation de CF₃CHFCH₂F se produit en présence d'hydrogène.

4. Procédé selon la revendication 1, dans lequel les composés métalliques sont des oxydes, des fluorures, et des oxyfluorures d'au moins un métal sélectionné parmi le groupe constitué du sodium, du potassium, du rubidium, du césium, de l'yttrium, du lanthane, du cérium, du praseodymium, du néodymium, du samarium, du chrome, du fer, du cobalt, du rhodium, du nickel, du cuivre, du zinc, et de leurs mélanges.

5. Procédé selon la revendication 1, dans lequel le catalyseur d'hydrogénation est un catalyseur de métal supporté sur du charbon.

6. Procédé selon la revendication 5, dans lequel le métal supporté sur du charbon est un métal du groupe VIII ou du rhénium.

7. Procédé selon la revendication 5, dans lequel le métal supporté sur du charbon est un catalyseur de palladium supporté sur du charbon.

8. Procédé selon la revendication 5, dans lequel le catalyseur de déshydrofluorination est constitué essentiellement d'alumine gamma, de fluorure d'aluminium, d'alumine fluorée, et de leurs mélanges.

9. Procédé selon la revendication 5, dans lequel le catalyseur de déshydrofluorination est constitué essentiellement d'oxyde de chrome.

10. Procédé de production de CF₃CF=CH₂ comprenant les étapes de:
(a) addition d'hydrogène et de CF₃CF=CHF à un récipient réactionnel contenant un catalyseur d'hydrogénation;
(b) réaction dudit CF₃CF=CHF avec de l'hydrogène sur ledit catalyseur d'hydrogénation pour produire du CF₃CHFCH₂F; et
(c) déshydrohalogénation de CF₃CHFCH₂F par la réaction avec une solution aqueuse alcaline en la présence d'un solvant non alcoolique non aqueux, et en présence d'un catalyseur de transfert de phase sélectionné parmi le groupe constitué des éthers couronnes, des sels d'onium, des cryptands, des polyalcylène glycols, et de leurs mélanges.

11. Procédé selon la revendication 10, dans lequel la solution aqueuse alcaline est fabriquée à partir d'une base sélectionnée parmi le groupe constitué de l'hydroxyde de lithium, de l'hydroxyde de sodium, de l'hydroxyde de potassium, de l'hydroxyde de calcium, de l'oxyde de magnésium, de l'oxyde de calcium, du carbonate de sodium, du carbonate de potassium, du phosphate de sodium, du phosphate de potassium, et de leurs mélanges.

12. Procédé selon la revendication 10, dans lequel le solvant est sélectionné parmi le groupe constitué des nitriles d'alkyle et d'aryle, des éthers d'alkyle et d'aryle, des amides, des cétones, des sulfoxydes, des esters de phosphate et de leurs mélanges.

13. Procédé selon la revendication 12, dans lequel le catalyseur de transfert de phase est soit le 18-couronne-6, le 15-couronne-5, un sel d'ammonium quaternaire soit leurs mélanges.

14. Procédé selon la revendication 13, dans lequel la base dans la solution aqueuse alcaline est l'hydroxyde de potassium et le catalyseur de transfert de phase est soit le 18-couronne-6, le chlorure de méthyltrioctylammonium soit leurs mélanges.
